(19) Europäisches Patentamt
European Patent Office
Office européen des brevets

(11) Veröffentlichungsnummer: **0 348 347**
**A2**

# (12) EUROPÄISCHE PATENTANMELDUNG

(21) Anmeldenummer: **89810446.8**

(22) Anmeldetag: **13.06.89**

(51) Int. Cl.[4]: **C 09 B 67/22**
C 09 B 67/10, C 09 B 67/04,
C 09 B 48/00

(30) Priorität: **20.06.88 US 209260**

(43) Veröffentlichungstag der Anmeldung:
**27.12.89 Patentblatt 89/52**

(84) Benannte Vertragsstaaten: **DE FR GB IT**

(71) Anmelder: **CIBA-GEIGY AG**
**Klybeckstrasse 141**
**CH-4002 Basel (CH)**

(72) Erfinder: **Jaffe, Edward E., Dr.**
**3 Crenshaw Drive**
**Wilmington Delaware 19810 (US)**

**Pfenninger, Johannes, Dr.**
**2233 Inwood Road**
**Wilmington Delaware 19810 (US)**

## (54) Verfahren zur Herstellung fester Lösungen von Chinacridonen.

(57) Verfahren zur Herstellung einer festen Lösung von mindestens zwei Chinacridonverbindungen durch Vermahlen der rohen oder subpigmentären Chinacridonverbindungen bei einer Temperatur zwischen 20 und 40° C in Gegenwart der 5- bis 20-fachen Gewichtsmenge, bezogen auf die Chinacridonmenge, eines niedrigsiedenden Alkohols ausgewählt aus der Gruppe bestehend aus $C_1$-$C_5$-Alkanolen und Glykolen, und von 0,5 bis 10,0 Gew.%, bezogen auf den Alkohol, einer Base ausgewählt aus der Gruppe bestehend aus Alkalimetallhydroxiden und quaternären Ammoniumhydroxiden,
und Isolieren der erhaltenen festen Lösung.

Dieses Verfahren erlaubt es feste Lösungen von hohem Kristallinitätsgrad mit ausgezeichneten Pigmenteigenschaften und hoher Hitzebeständigkeit bei oder annähernd bei Zimmertemperatur herzustellen.

EP 0 348 347 A2

**Beschreibung**

**Verfahren zur Herstellung fester Lösungen von Chinacridonen**

Die Verbindungen der Chinacridon-Serie und ihre Pigmenteigenschaften sind durch die Beschreibungungen in zahlreichen Patenten und in der technischen Literatur sehr wohl bekannt. Auch feste Lösungen von Chinacridonen sind bekannt. Diese zeichnen sich aus durch ihre guten Pigmenteigenschaften und insbesondere durch die Kombination von guter Lichtbeständigkeit und Hitzebeständigkeit. Die festen Lösungen werden durch Röntgenbeugungsdiagramme charakterisiert, die sich von denjenigen einer physikalischen Mischung derselben Komponenten in ähnlichen Konzentrationen unterscheiden.

Feste Lösungen von Chinacridonen sind beispielsweise in der US-PS 3 160 510 beschrieben. Sie werden dadurch hergestellt, dass eine Dimethylformamidsuspension der Ausgangsprodukte eine Stunde am Rückfluss erhitzt wird. Die US-PS 3 607 336 beschreibt ein Verfahren, bei welchem die rohen Komponenten in konzentrierter Schwefelsäure gelöst, durch eingiessen in hoch turbulentem Wasser wieder ausgefällt werden und das erhaltene Produkt danach in verdünnter Schwefelsäure erhitzt wird. Weitere Herstellungsmethoden von festen Lösungen sind auch in der US-PS 3 681 100 und der US-PS 3 686 009 beschrieben. Im allgemeinen handelt es sich um mehrstufige Verfahren, die bei hohen Temperaturen und in saurem Medium durchgeführt werden. Erwünscht sind aber einfachere, ökonomischere Verfahren bei Raumtemperatur.

Es wurde nun gefunden, dass überraschenderweise feste Lösungen von Chinacridonen mit guten Pigmenteigenschaften und hohem Kristallinitätsgrad erhalten werden können, wenn man mindestens zwei rohe Chinacridonverbindungen bei oder annähern bei Zimmertemperatur in einer genügenden Menge eines niedrigsiedenden Alkohols in Gegenwart einer Base vermahlt. Dieses Verfahren zeigt eine grosse Flexibilität. Eine grosse Auswahl verschiedener fester Lösungen kann hergestellt werden, die sich bezüglich Zusammensetzung, Konzentration der Komponenten und der Partikelgrösse unterscheiden. Man kann somit Pigmente hoher Deckkraft als auch solche mit mittlerer bis hoher Transparenz herstellen. Rohe, vorvermahlene oder mit Säure vorbehandelte Chinacridone können als Ausgangsprodukte für das erfindungsgemässe Mahlverfahren verwendet werden. Im allgemeinen wird das Pigment aus der Mahlaufschlämmung direkt mit der geeigneten Partikel grösse isoliert. Die erhaltenen festen Lösungen zeichnen sich durch ihre guten Pigmenteigenschaften aus, insbesondere durch gute Licht- und Wetterbeständigkeit, Beständigkeit gegen verschiedene Lösungsmittel, was sie sehr geeignet für den Einsatz in Lacken, insbesondere in Automobillacken macht. Sie zeigen auch eine hohe Hitzebeständigkeit und sind deshalb sehr gut geeignet für den Einsatz in verschiedenen Polymeren.

Die vorliegende Anmeldung betrifft somit ein Verfahren zur Herstellung einer festen Lösung von mindestens zwei Chinacridonverbindungen durch Vermahlen der rohen oder subpigmentären Chinacridonverbindungen bei einer Temperatur zwischen 20 und 40°C in Gegenwart der

5- bis 20-fachen Gewichtsmenge, bezogen auf die Chinacridonmenge, eines niedrigsiedenden Alkohols ausgewählt aus der Gruppe bestehend aus $C_1$-$C_5$-Alkanolen und Glykolen, und von

0,5 bis 10,0 Gew.%, bezogen auf den Alkohol, einer Base ausgewählt aus der Gruppe bestehend aus Alkalimetallhydroxiden und quaternären Ammoniumhydroxiden,

und Isolieren der erhaltenen festen Lösung.

Die Komponenten der festen Lösung können Chinacridon und seine Derivate, wie z.B. 4,11-Dichlorchinacridon, 2,9-Dichlorchinacridon, 4,11-Difluorchinacridon, 2,9-Difluorchinacridon, 2,9-Dimethylchinacridon und Gemische davon sein.

Im allgemeinen handelt es sich z.B. um lineare Chinacridone der Formel

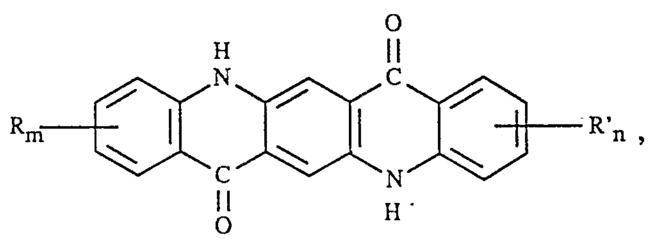

worin R und R′, unabhängig voneinander, Fluor, Chlor, Brom, $C_1$-$C_4$-Alkyl, $C_1$-$C_4$-Alkoxy und m und n, unabhängig voneinander, eine Zahl von Null bis 2 bedeuten, oder um Isochinacridone der Formel

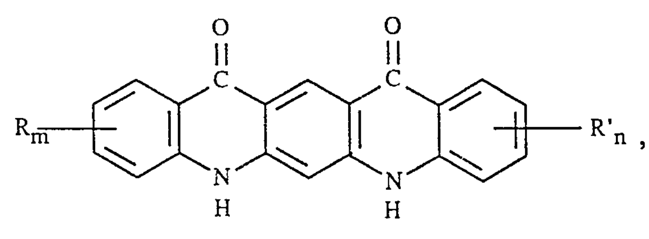

worin R, R′, m und n die oben angegebene Bedeutung haben.

$C_1$-$C_4$-Alkyl bedeutet z.B. Methyl, Ethyl, n-Propyl, Isopropyl, n-Butyl, sec.-Butyl oder tert.-Butyl.

$C_1$-$C_4$-Alkoxy bedeutet z.B. Methoxy, Ethoxy, n-Propoxy, Isopropoxy, n-Butoxy, sec.-Butoxy oder tert.-Butoxy.

Bevorzugt ist eine der Chinacridonkomponenten ein unsubstituiertes Chinacridon.

Einige der bevorzugten festen Lösungen, die nach dem erfindungsgemässen Verfahren hergestellt werden können sind die folgenden:

1) Feste Lösungen enthaltend zwei Komponenten ausgewählt aus der Gruppe bestehend aus unsubstituiertem Chinacridon und durch den gleichen Substituent F, Cl, Br, $CH_3$ oder $0CH_3$ 4,11-disubstituierten Chinacridonen.

2) Feste Lösungen enthaltend zwei Komponenten ausgewählt aus der Gruppe bestehend aus unsubstituiertem Chinacridon und durch den gleichen Substituent F, Cl, Br, $CH_3$ oder $0CH_3$ 2,9-disubstituierten Chinacridonen.

3) Feste Lösungen enthaltend zwei Komponenten ausgewählt aus der Gruppe bestehend aus unsubstituiertem Isochinacridon und symmetrisch durch F, Cl, Br, $CH_3$ oder $OCH_3$ disubstituierten Isochinacridonen.

Besonders bevorzugte feste Lösungen erhält man mit 60 Gew.% Chinacridon und 40 Gew.% 2,9-Dichlorchinacridon (brillantes Magenta) oder mit 60 Gew.% Chinacridon und 40 Gew.% 4,11-Dichlorchinacridon (brillantes Scharlach). Gewünschtenfalls kann die Chinacridonkonzentration bis z.B. auf 10 Gew.% reduziert werden, wie beispielsweise in der festen Lösung aus 10 Gew.% Chinacridon und 90 Gew.% 2,9-Dichlorchinacridon.

Das erfindungsgemässe Verfahren ist besonders geeignet zur Herstellung von deckenden scharlachfarbenen festen Lösungen aus Chinacridon und 4,11-Dichlorchinacridon. Eine sehr wertvolle feste Lösung kann beispielsweise aus den zwei Rohpigmenten durch Mahlung von 60 Gew.% Chinacridon und 40 Gew.% 4,11-Dichlorchinacridon in Gegenwart von Alkohol und Base nach dem erfindungsgemässen Verfahren erhalten werden. Durch Zugabe eines wachstumshemmenden Mittels, wie nachstehend beschrieben, kann ein entsprechendes transparentes Produkt erhalten werden. Wenn erwünscht, können auch 60 Gew.% 4,11-Dichlorchinacridon und 40 Gew.% Chinacridon eingesetzt werden, wobei die Farbe nach kürzeren Wellenlängen verschoben wird. Im allgemeinen beträgt das Verhältnis von Chinacridon zu substituiertem Chinacridon 95:5 bis 5:95, insbesondere aber 75:25 bis 25:75.

Beim erfindungsgemässen Verfahren verfährt man im allgemeinen derart, dass man die vorgemahlenen oder mit Säure vorbehandelten (acid pasted) Chinacridone, den Alkohol und die Base zusammen mit den Mahlhilfsmitteln in eine geeignete Mühle bei einer Temperatur zwischen 20 und 40°C, vorzugsweise bei Raumtemperatur während 24 bis 96 Stunden vermahlt und anschliessend die erhaltene feste Lösung isoliert. Der Alkohol muss in Gegenwart der Base stabil sein. Bei den $C_1$-$C_5$-Alkanolen kann es sich um geradkettige oder auch um verzweigte Alkylderivate handeln. Bevorzugt werden Ethanol, Butanol, Pentanol und insbesondere Methanol. Bevorzugter Glykol ist Ethylenglykol. Alkalimetallhydroxide sind beispielsweise Natrium-, Kalium- und Lithiumhydroxid. Ein quaternäres Ammoniumhydroxid ist z.B. Benzyltrimethylammoniumhydroxid. Natrium- und Kaliumhydroxid werden bevorzugt.

Ueberraschenderweise erlaubt das erfindungsgemässe Mahlverfahren die Herstellung von festen Lösungen mit hohem Kristallinitätsgrad aus Chinacridonen bei Raumtemperatur, im Gegensatz zu den bekannten Verfahren, die eine Behandlung bei hoher Temperatur erfordern. Ausserdem, da meistens die gewünschte Partikelgrösse beim Mahlvorgang erhalten wird, ist eine zusätzliche Hitzebehandlung nicht unbedingt notwendig.

Wie bereits beschrieben, können rohe Chinacridone oder Chinacridone in subpigmentärer Form, die z.B. durch Vorvermahlen oder durch Vorbehandlung mit Säure (acid pasting) hergestellt werden, im erfindungsgemässen Verfahren eingesetzt werden.

Unter Vorvermahlen, gemäss der vorliegenden Erfindung, versteht man ein Mahlen unter Ausschluss von Flüssigkeiten oder, wenn Flüssigkeiten, wie z.B. phasenlenkende Lösungsmittel oder oberflächenaktive Mittel, verwendet werden, dann nur in so kleinen Mengen (höchstens 10,0 Gew.%, bezogen auf das Pigment) oder von solcher Beschaffenheit, dass das Pigment beim Mahlen als Pulver vorliegt. Die Vorvermahlung wird gegebenenfalls in Gegenwart einer kleinen Menge, 10 Gew.% oder weniger, eines Salzes, wie z.B. wasserfreies Natriumsulfat, durchgeführt. Durch die Anwesenheit des Natriumsulfats wird beispielsweise die Explosionstendenz des erzeugten Mahlpulvers unterbunden. Die Vorvermahlung kann in Gegenwart von verschiedenen Mahlhilfsmitteln, z.B. von Stahlkugeln, von Nägeln, von Stahlschrot, von Keramikkugeln oder -perlen durchgeführt werden. Wird die Vorvermahlung mit Mahlhilfsmitteln aus Stahl durchgeführt, so ist am Ende des Mahlvorgangs eine Extraktion mit verdünnter Mineralsäure zur Entfernung des Metallabriebs wünschenswert.

Unter Vorbehandlung mit Säure (acid pasting) versteht man die Auflösung des rohen Pigments in konzentrierter Schwefelsäure und das darauffolgende Einströmenlassen dieser Lösung in Wasser unter hohen Turbulenzbedingungen, zur Bildung subpigmentärer Partikel.

Für den erfindungsgemässen Alkohol/Base-Mahlvorgang eignen sich verschiedene Mahlhilfsmittel, wie z.B. Stahlschrot oder Keramikperlen. Letztere sind in verschiedenen Grössen und Zusammensetzungen erhältlich. Bevorzugt werden Keramikperlen von 1,6 bis 2,5 oder 2,5 bis 3,15 mm Durchmesser, die aus einer kristallinen Zirkonium- und einer amorphen Kieselsäurephase der entsprechenden Oxide hergestellt werden. Zirkoniumoxidperlen die einen kleinen Anteil an Magnesiumoxid enthalten sind ebenfalls geeignet. Werden Mahlhilfsmittel aus Stahl eingesetzt, so ist, wie oben bereits erwähnt eine Extraktion mit verdünnter Mineralsäure zur Entfernung des Mahlabriebs am Ende des Mahlvorganges wünschenswert.

Der Alkohol wird bevorzugt in einer 12- bis 20-fachen Menge, bezogen auf das Gewicht des Pigments eingesetzt. Die Base wird zweckmässig als wässrige Lösung und bevorzugt in einer Menge von 1,0 bis 5,0 Gew.%, bezogen auf den Alkohol, zugesetzt.

Ist die erforderliche Menge an Base nicht vorhanden, so ist das Wachstum der Partikel minimal und die Bildung der festen Lösung unvollständig. Bei der angegebenen Basenkonzentration ist keine Bildung des Kaliumsalzes des Chinacridons (welches das Partikel wachstum beeinflusst) ersichtlich, das wegen seiner deutlich blauen Farbe leicht erkennbar wäre.

Das Partikelwachstum kann im erfindungsgemässen Alkohol/Base-Mahlverfahren durch den Zusatz eines wachstumshemmenden Mittels kontrolliert werden. Die Partikelgrösse kann damit in einem breiten Bereich, von 0,01 µ bis 0,8µ d.h. von hochtransparenten bis deckenden Pigmenten, eingestellt werden. Geeignet zu diesem Zweck sind z.B. 2-Phthalimidochinacridon, die Salze der Chinacridonsulfosäure und andere ähnliche Derivate. Das wachstumshemmende Mittel wird im allgemeinen in Mengen von 0,5 bis 10, bevorzugt 1 bis 5 Gew.%, bezogen auf das Pigment eingesetzt. 2-Phthalimidomethylchinacridon der Formel

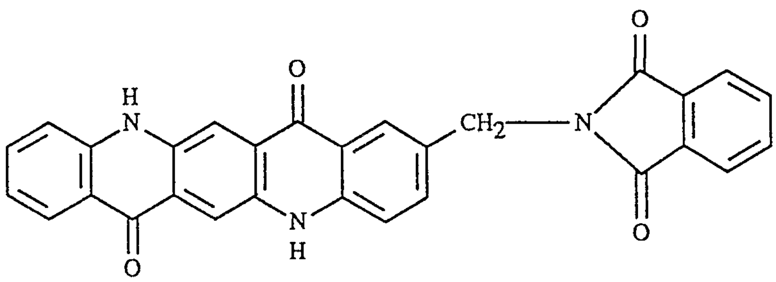

ist als wachstumshemmendes Mittel im erfindungsgemässen Alkohol/Base-Mahlverfahren ganz besonders geeignet, da es im basischen Medium eine Ringöffnung zum Salz der folgenden Formel

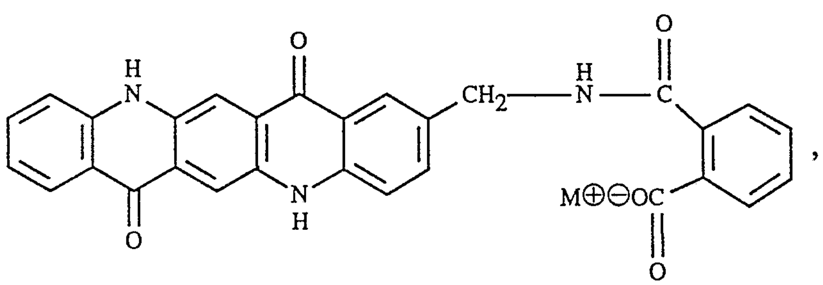

worin M⊕ Li⊕, Na⊕, K⊕ oder $NH_4$⊕ bedeutet, eingeht, welches eine stärkere wachstumshemmende Wirkung hat, als das unter sauren oder neutralen Bedingungen vorhandene "Imid" mit geschlossenem Ring.

Wenn erwünscht können auch oberflächenaktive Mittel oder Verdünnungsmittel zugesetzt werden, unter der Bedingung, dass diese Additive durch das basische Medium nicht desaktiviert werden. Der einfache Zusatz von anionischen, kationischen oder nichtionogenen Tensiden kann, wenn die Materialien nicht wasserlöslich sind, die Pigmenteigenschaften der erfindungsgemäss hergestellten festen Lösungen noch zusätzlich verbessern.

Obschon die endgültige Partikelgrösse während des Nassmahlvorganges erzeugt worden ist und deshalb das Produkt direkt aus der Mahlaufschlämmung nach Entfernung der Mahlhilfsmittel isoliert werden könnte, wird das Pigment am besten dadurch isoliert, dass man die Pigmentaufschlämmung von den Mahlhilfsmitteln durch Verdünnung und Auswaschen mit Alkohol und/oder Wasser befreit und anschliessend den Alkohol abdestilliert. Der Alkohol kann somit zurückgewonnen werden und das Pigment durch Filtrieren der zurückgebliebenen nichtbrennbaren Aufschlämmung und Neutralwaschen mit Wasser isoliert werden. Die erhaltenen Pigmente zeichnen sich, wie durch Röntgenbeugungsanalyse bestätigt wird, durch eine hervorragende Kristallinität aus.

Wie viele andere Pigmente können die erfindungsgemäss hergestellten festen Lösungen einer der üblichen Oberflächenbehandlungen zur Verbesserung ihrer Anwendungseigenschaften in Automobil- oder anderen Lacksystemen unterworfen werden. Additive, die die Flokkulation vermindern oder vermeiden und die Dispersionsbeständigkeit der Pigmente erhöhen, können vorteilhaft eingesetzt werden. So behandelte Pigmente zeichnen sich allein oder in Mischungen durch ausserordentliche Eigenschaften in verschiedenen Lacksystemen, z.B. Automobillacke, wie Acryl-, Alkyd- und Polyesterlacke, sowie in anderen Lacksystemen aus. 2-Phthalimidomethylchinacridon, Chinacridonsulfosäure und andere ähnliche Derivate sind als Antiflokkungsmittel geeignet. In bestimmten Systemen können die Pigmenteigenschaften auch durch Zusatz von polymeren Dispersionsmitteln erhöht werden.

Bei der erfindungsgemäss erhältlichen festen Lösung handelt es sich um ein Pigment, das sich

beispielsweise als Pulver, Teig, Flushpaste und Zubereitung anwenden lässt und sich z.B. für Druckfarben, Leimfarben, Binderfarben oder Lacke aller Art, wie physikalisch und oxydativ trocknende Lacke, säure-, amin- und peroxidhärtende Lacke oder Polyurethanlacke eignet. Das Pigment kann auch zum Färben von synthetischen, halbsynthetischen oder natürlichen makromolekularen Stoffen verwendet werden, wie Polyvinylchlorid, Polystyrol, Polyäthylen, Polyestern, Phenoplasten, Aminoplasten und Gummi. Weitere Anwendungsgebiete sind das Färben von natürlichen, regenerierten oder künstlichen Fasern, wie Glas-, Silikat-, Asbest-, Holz-, Cellulose-, Acetylcellulose-, Polyacrylnitril-, Polyester-, Polyurethan- und Polyvinylchloridfasern oder deren Gemische, eventuell zusammen mit anderen organischen oder anorganischen Pigmenten. Man erhält mit dem neuen Pigment Drucke, Lackierungen, Anstriche, Beläge, Beschichtungen, geformte Gebilde, wie Folien, Fäden, Platten, Blöcke, Granulate und Stäbe, mit brillanter roter Farbe von hervorragender Dauerhaftigkeit.

Die erfindungsgemäss erhältlichen Pigmente können zum Färben von festen, elastischen, pastenartigen, dickflüssigen, dünnflüssigen oder thixotropen Massen verwendet und in diese nach an sich bekannten Verfahren eingearbeitet werden. Wasserhaltige Teige können beispielsweise durch Einrühren des Pigments in Wasser, gegebenenfalls unter Zusatz eines Netz- oder Dispergiermittels oder durch Einrühren oder Einkneten des Pigments in ein Dispergiermittel in Gegenwart von Wasser und gegebenenfalls von organischen Lösungsmitteln oder Oelen erhalten werden. Diese Teige können beispielsweise wiederum zur Herstellung von Flushpasten, Druckfarben, Leimfarben, Kunststoffdispersionen und Spinnlösungen verwendet werden. Das Pigment kann aber auch durch Einrühren, Einwalzen, Einkneten oder Einmahlen in Wasser, organische Lösungmittel, nicht trocknende Oele, trocknende Oele, Lacke, Kunststoffe oder Gummi gebracht werden. Schliesslich ist es auch möglich, das Pigment durch trockenes Mischen mit organischen oder anorganischen Massen, Granulaten, Faserstoffen, Pulvern und anderen Pigmenten zu Stoffmischungen zu verarbeiten.

Die erfindungsgemäss erhältlichen Pigmente zeichnen sich nicht nur durch hohe Kristallinität, gute Allgemeinechtheiten, wie Licht- und Wetterbeständigkeit und Unempfindlichkeit gegen Lösungsmittel und Weichmacher, sondern auch durch ihre vorzügliche Hitzebeständigkeit, aus. Dadurch können die erfindungsgemäss erhältlichen Pigmente in Polyethylen hoher oder niedriger Dichte oder in Polypropylen eingearbeitet werden, ohne dass die Farbe durch die hohen Verarbeitungstemperaturen abgestumpft wird.

Die nachfolgenden Beispiele erläutern die Erfindung.

Beispiel 1: Herstellung einer festen Lösung aus Chinacridon und 4,11-Dichlorchinacridon.

In eine handelsübliche Mühle (2,84 l) werden 2500 g Keramikperlen von 1,6-2,5 mm Durchmesser, bestehend im Durchschnitt aus ca. 69 % $ZrO_2$ und 31 % $SiO_2$, 30,5g rohes γ-Chinacridon, 19,5g rohes 4,11-Dichlorchinacridon, 791 g Methanol und 75 g 44%-ige wässrige Kalilauge gegeben. Die Mühle wird 72 Stunden bei 30°C mit 68 U/Min., d.h. ca. 74% der kritischen Geschwindigkeit gedreht ("kritische Geschwindigkeit" ist die Geschwindigkeit, bei der die Fliehkraft die Schwerkraft aufhebt, so dass die Mahlhilfsmittel gegen die Wand der Mühle gehalten werden).

Die Pigmentaufschlämmung wird von den Keramikperlen getrennt und letztere mit Methanol gewaschen. Die kombinierten Aufschlämmungen werden dampfdestilliert bis die Temperatur der wässrigen Pigmentsuspension 95°C erreicht. Diese Temperatur wird 10 Minuten beibehalten, dann wird das Pigment durch Filtrieren isoliert, mit Wasser neutral gewaschen und getrocknet. Man erhält 48,5g eines Pigments mit einer spezifischen Oberfläche von 25,6 $m^2/g$. Die Röntgenbeugungsanalyse zeigt einen hohen Kristallinitätsgrad.

Nach üblichen Methoden in einem Oeldruckfarben-System eingearbeitet (z.B. durch Ausreiben auf einem Hoover-Muller in lithographischem Firnis), ergibt das Pigment Ausfärbungen, die sich insbesondere durch die Farbstärke und Deckkraft im Vollton auszeichnen.

Farbstärke und Deckkraft im Vollton sind, insbesondere bei der Zubereitung von Automobillacken, sehr begehrte Eigenschaften.

Ein praktisch identisches Produkt, wie durch Röntgenbeugungsanalyse bestätigt, erhält man, wenn man die Pigmentaufschlämmung nach der Abtrennung der Mahlhilfsmittel filtriert und das Pigment auswäscht, ohne Destillationsvorgang, d.h. ohne Hitzebehandlung. Dies unterstützt die Behauptung, dass ein Isolierverfahren (z.B. Dampfdestillation oder Extraktion mit Säure) für die Bildung der festen Lösung nicht wesentlich ist.

Wird das gleiche Mahlverfahren mit Methanol, aber ohne Zusatz der Base durchgeführt, so ist das erhaltene Produkt völlig verschieden. Im Röntgenbeugungsdiagramm erkennt man die verschiedenen Phasen der Ausgangsprodukte und beim Ausreiben in lithographischem Firnis erhält man Ausfärbungen die im Vollton dunkler und im Verschnitt blauer sind.

Werden die gleichen Mengen an rohen Pigmenten, Methanol und Kalilauge mehrere Stunden am Rückfluss gekocht ohne zu mahlen, so wird keine feste Lösung erhalten und beim Ausreiben des erhaltenen Produktes in lithographischem Firnis erhält man ebenfalls, wie beim Auslassen der Base, Ausfärbungen, die im Vollton dunkler und im Verschnitt blauer sind.

Wird das Produkt von Beispiel 1 nach allgemein üblichen Methoden in einem Alkydlacksystem eingearbeitet, so zeigen die damit erhaltenen Lackierungen auf Aluminiumblech eine hervorragende Wetterbeständigkeit. Wird eine normale physikalische Mischung von 4,11-Dichlorchinacridon und Chinacridon im gleichen Alkydlacksystem eingearbeitet, so ergeben die damit erhaltenen Lackierungen im Wetterbeständigkeitstest eine deutliche Verfärbung.

Wird das Pigment von Beispiel 1 nach allgemein üblichen Methoden in Polypropylen eingearbeitet, so zeigt es eine ausgezeichnete Hitzebeständigkeit bis 300°C. Das Pigment von Beispiel 1 eignet sich auch sehr gut

zum Färben von Polyethylen hoher Dichte, wobei eine weit höhere Verzugsfreiheit gewährleistet wird, als beim Einsatz von nach anderen Verfahren hergestellten Pigmenten.

Beispiel 2: Herstellung einer festen Lösung kleinerer Partikelgrösse aus Chinacridon und 4,11-Dichlorchinacridon.

In eine Pilot-Plant-Mühle werden 454 kg Stahlzylinder (Cly-Pebs) von 1,27 cm Durchmesser und 1,27 cm Länge, 45,4 kg Nägel von 10 cm Länge (Twenty Penny), 10,896 kg rohes γ-Chinacridon, 7,264 kg rohes 4,11-Dichlorchinacridon und 2,270 kg wasserfreies Natriumsulfat gegeben. Die Mühle wird 50 Stunden bei 47-48° C mit 40 U/Min., d.h. mit ca. 74 % der kritischen Geschwindigkeit gedreht. Danach wird das Mahlgut durch Filtrieren durch ein Sieb von Zylindern und Nägeln abgetrennt.

132 g des erhaltenen gemahlenen Pulvers werden in eine handelsübliche Mühle (2,84 l) mit 4800 g Stahlschrot (3,2 mm Ø), 791 g Methanol, 75 g 50%-igem wässrigem Natriumhydroxid und 2,4g 2-Phthalimidomethylchinacridon gegeben. Die Mühle wird 72 Stunden bei etwa 30° C mit 68 U/Min. gedreht. Die Pigmentaufschlämmung wird von den Mahlhilfsmitteln abgetrennt und letztere werden mit Methanol gewaschen. Die Aufschlämmung wird in einem Vierhalskolben mit Rührer, Thermometer, Dampfeinleitungsrohr und Kühler dampfdestilliert. Dabei wird der Methanol zusammen mit etwas Wasser abdestilliert bis die Temperatur auf 95° C steigt. Diese Temperatur wird etwa 10 Minuten gehalten, dann wird die Aufschlämmung auf 80° C abgekühlt, mit verdünnter Schwefelsäure auf pH 1,5 angesäuert und bei 80-85° C eine Stunde gerührt. Dann werden 24 g Kristallwasser enthaltendes Aluminiumsulfat zugegeben, gefolgt, durch regelmässige Zugabe während 30 Minuten, von 3,14 g Chinacridon-2-sulfosäure. Nach Beendigung der Zugabe wird die Temperatur 30 Minuten bei 80-85° C gehalten. Das Pigment wird abfiltriert und mit heissem Wasser neutral gewaschen. Nach dem Trocknen bei 80° C erhält man 111,4g Pigment mit einer spezifischen Oberfläche von 66,9 $m^2/g$. Das Röntgenbeugungsdiagramm des erhaltenen Produktes zeigt die gleichen Linien wie dasjenige des Produktes von Beispiel 1.

Bei Ausreiben in lithiographischem Firnis ergibt das Pigment intensive transparente Ausfärbungen im Vollton und eine farbstarke Scharlachausfärbung im Verschnitt mit Zinkoxid.

Wird im Alkohol/Base-Mahlvorgang die Zugabe von 2-Phthalimidomethylchinacridon unterlassen, so erhält man ein Produkt mit relativ grosser Partikelgrösse (spezifische Oberfläche 31,2 $m^2/g$), das schwache und deckende Ausfärbungen im Vollton ergibt. Wird zusätzlich die Pigmentaufschlämmung vor der Dampfdestillation 3 Stunden am Rückfluss erhitzt, so erhält man ein Produkt mit einer noch grösseren Partikelgrösse (spezifische Oberfläche 27,6 $m^2/g$) das etwas schwächere Ausfärbungen ergibt. Dies zeigt, dass beim Rückflusskochen im basischen Medium Partikelwachstum stattfindet.

Beispiel 3: Herstellung einer relativ deckenden festen Lösung aus 60 % γ-Chinacridon und 40 % 2,9-Dichlorchinacridon.

In eine Labormühle werden 1500 g Stahlkugeln (1,27 Ø), 150 g Zimmerer-Nägel, 30 g rohes γ-Chinacridon, 20 g rohes 2,9-Dichlorchinacridon und 5 g wasserfreies Natriumsulfat gegeben. Die Mühle wird 24 Stunden mit ca. 75 % der kritischen Geschwindigkeit bei Zimmertemperatur gedreht. Die Kugeln und Nägel werden mittels eines Siebes abgetrennt und das trockene Mahlpulver isoliert.

In eine 236 ml-Mühle werden 300 g Keramikperlen (wie in Beispiel 1), 6g des wie oben beschrieben erhaltenen Mahlpulvers, 79 g Methanol und 5,4 g 50%-iges wässriges Natriumhydroxid gegeben. Die Mühle wird 72 Stunden bei 30° C mit ca. 74 % der kritischen Geschwindigkeit gedreht. Die Pigmentaufschlämmung wird dann wie in Beispiel 1 beschrieben aufgearbeitet und man erhält 4,2 g Pigment hoher Kristallinität (gemäss Röntgenbeugungsdiagramm) mit einer spezifischen Oberfläche von 48,3 $m^2/g$.

Ein praktisch identisches Produkt, welches vollständig als feste Lösung vorliegt, erhält man auch ohne Behandlung in der Hitze (Dampfdestillation), durch einfaches Filtrieren der Pigmentaufschlämmung und Auswaschen des Pigments nach Abtrennung von den Mahlhilfsmitteln.

Beim Ausreiben in lithographischem Firnis ergeben diese Pigmente eine helle Ausfärbung im Vollton und eine intensiv bläulich-rote Ausfärbung im Verschnitt.

Beispiel 4: Herstellung einer hochkristallinen festen Lösung kleiner Partikelgrösse aus Chinacridon und 2,9-Dichlorchinacridon.

Eine Mischung aus 60 % rohem γ-Chinacridon und 40 % 2,9-Dichlorchinacridon wird in Gegenwart von Natriumsulfat einer Trockenmahlung, wie in Beispiel 2 beschrieben, unterworfen. Danach werden 100 g des so erhaltenen Mahlpulvers in eine handelsübliche Mühle (2,84 l) zusammen mit 4800 g Stahlschrot, 791 g Methanol, 75 g 50%-igem wässrigem Natriumhydroxid und 4,5 g 2-Phthalimidomethylchinacridon gegeben. Vermahlung und Aufbereitung erfolgen wie in Beispiel 2 beschrieben, mit der Ausnahme, dass die Oberflächenbehandlung des Pigments proportional zur eingesetzten Pigmentmenge ist (d.h. das 100 g kristallines Aluminiumsulfat dazu eingesetzt werden). Nach der Trocknung erhält man 89 g Pigment als feste Lösung mit hohem Kristallinitätsgrad (gemäss Röntgenbeugungsdiagramm) mit einer spezifischen Oberfläche von 78,6 $m^2/g$.

Beim Ausreiben in lithographischem Firnis ergibt das Pigment eine intensive transparente Magenta-Ausfärbung im Vollton und eine relativ starke, bläulich-rote Ausfärbung im Verschnitt mit Zinkoxid.

Wird die Zugabe von 2-Phthalimidomethylchinacridon unterlassen, so erhält man ein Produkt mit einer spezifischen Oberfläche von 49,8 $m^2/g$, das relativ deckende Ausfärbungen ergibt. Es ist hell im Vollton,

schwächer und etwas gelber im Zinkoxid-Verschnitt. Das Produkt ist im Wesentlichen gleich, wie das Produkt von Beispiel 3.

Beispiel 5: Herstellung einer festen Lösung relativ grosser Partikelgrösse aus Chinacridon und 2,9-Dimethylchinacridon.

In eine Labormühle werden 150 g Stahlkugeln (1,27 cm ∅), 150 g Zimmerer-Nägel, 30 g rohes γ-Chinacridon, 30 g rohes 2,9-Dimethylchinacridon und 5 g wasserfreies Natriumsulfat gegeben. Die Mühle wird 48 Stunden mit ca. 75 % der kritischen Geschwindigkeit gedreht. Kugeln und Nägel werden mittels eines Siebes abgetrennt und das trockene Mahlpulver isoliert.

50 g des so erhaltenen Mahlpulvers werden in eine Labormühle (2,84 l) zusammen mit 2500 Keramikperlen (wie in Beispiel 1), 791 g Methanol und 18,8 g 50%-igem wässrigem Natriumhydroxid gegeben. Die Mühle wird 72 Stunden bei ca. 30° C mit ca. 74 % der kritischen Geschwindigkeit gedreht. Die Keramikperlen werden mittels eines Keramiksiebes abgetrennt und mit 632 g Methanol gewaschen, so dass praktisch das ganze Pigment als Aufschlämmung vorliegt. Diese Aufschlämmung wird in einem 4-Halskolben mit Rührer, Thermometer, Dampfeinleitungsrohr und Kühler dampfdestilliert. Dabei wird der Methanol zusammen mit etwas Wasser abdestilliert bis die Temperatur 95° C erreicht.

Die Aufschlämmung wird auf 80° C abgekühlt und mit 20%-iger Schwefelsäure auf pH 1,5 angesäuert. Dann wird sie auf 95° C erhitzt und eine Stunde bei dieser Temperatur gehalten. Danach wird das Produkt durch Filtrieren isoliert, mit heissem Wasser neutral gewaschen und bei 80° C getrocknet. Man erhält 42,8g eines Produktes, welches in lithographischem Firnis ausgerieben eine relativ helle, deckende Ausfärbung ergibt. Gemäss Röntgenbeugungsdiagramm handelt es sich um eine feste Lösung von hohem Kristallinitätsgrad.

Beispiel 6: Herstellung einer festen Lösung aus 90 % 2,9-Dichlorchinacridon und 10 % Chinacridon.

Eine Mischung aus 90 % rohem 2,9-Dichlorchinacridon und 10 % rohem γ-Chinacridon wird in Gegenwart von Natriumsulfat einer Trockenmahlung, wie in Beispiel 3 beschrieben, unterworfen. 50 g des so vorvermahlenen Pulvers werden in eine Labormühle (2,84 l) zusammen mit 2500 g Keramikperlen (wie in Beispiel 1), 791 g Methanol und 75 g 50%-igem wässrigem Natriumhydroxid gegeben. Vermahlung und Aufbereitung erfolgen wie in Beispiel 5 beschrieben. Man erhält 42,5g eines Pigments mit einer spezifischen Oberfläche von 59,0 $m^2/g$, welches eine relativ deckende Magenta-Ausfärbung ergibt und, gemäss Röntgenbeugungsdiagramm, einen hohen Kristallinitätsgrad aufweist.

Beispiel 7: Vorteilhafte Wirkung der Oberflächenbehandlung auf die rheologischen Eigenschaften.

91 g des Produktes von Beispiel 1, 3 g 2-Phthalimidomethylchinacridon und 6 g eines polymeren Dispergiermittels (erhalten durch Isolierung des trockenen Polymeren von ®Disperbyk 160 (BYK-Chemie)) werden zu einem homogenen Pulver vermischt. Wird diese Pigmentzusammensetzung nach üblichen Methoden in einem Alkydlacksystem eingearbeitet, so ergibt sich eine bedeutende Verbesserung der Viskosität der Dispersion sowie des Glanzes und des DOI (distinctues of image) der damit erhaltenen Lackierungen im Vergleich zum unbehandelten Pigment des Beispiels 1.

| | Viskosität* in mPa.s | Vollton** | |
|---|---|---|---|
| | | Glanz bei 20°/20° | DOI |
| Produkt dieses Beispiels | 60 | 90 | 95 |
| Unbehandeltes Produkt gemäss Beispiel 1 | 720 | 82 | 70 |

* Gemessen mit ®Brookfield Viskosimeter RVTD bei 10 U/Min. und 23°C.
** Volltonapplikation auf Aluminiumplatte (mit einer grauen Acrylgrundierung, die mit einer solchen Schicht überlackiert wird, dass über einem Schwarz/Weiss-Karton visuell eine vollständige Deckfähigkeit gewährleistet wird). Der Glanz wird bei 20°/20° mit einem Glanzmessgerät ®Glossguard G67526 (System 20/60/85) gemessen. Das DOI mit einem DOI-Meter der Firma Paul Gardner Co., Inc..

**Patentansprüche**

1. Verfahren zur Herstellung einer festen Lösung von mindestens zwei Chinacridonverbindungen durch Vermahlen der rohen oder subpigmentären Chinacridonverbindungen bei einer Temperatur zwischen 20 und 40°C in Gegenwart der5- bis 20-fachen Gewichtsmenge, bezogen auf die Chinacridonmenge, eines niedrigsiedenden Alkohols ausgewählt aus der Gruppe bestehend aus $C_1$-$C_5$-Alkanolen und Glykolen, und von
0,5 bis 10,0 Gew.%, bezogen auf den Alkohol, einer Base ausgewählt aus der Gruppe bestehend aus Alkalimetallhydroxiden und quaternären Ammoniumhydroxiden,
und Isolieren der erhaltenen festen Lösung.

2. Verfahren gemäss Anspruch 1, dadurch gekennzeichnet, dass es sich bei den genannten Chinacridonverbindungen um lineare Chinacridone der Formel

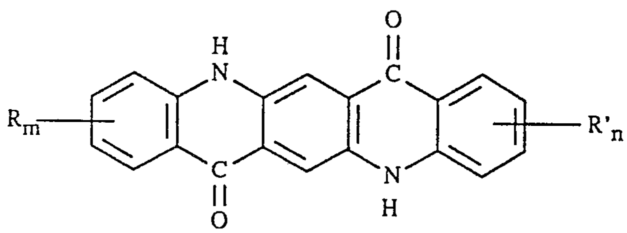 ,

worin R und R′, unabhängig voneinander, Fluor, Chlor, Brom, $C_1$-$C_4$-Alkyl, $C_1$-$C_4$-Alkoxy und m und n, unabhängig voneinander, eine Zahl von Null bis 2 bedeuten,
oder um Isochinacridone der Formel

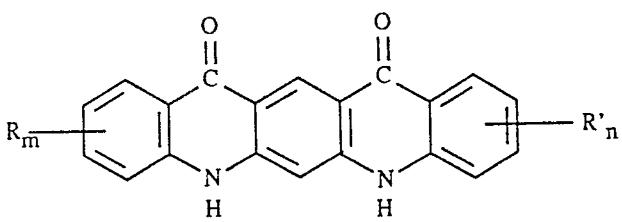 ,

worin R, R', m und n die oben angegebene Bedeutung haben.

3. Verfahren gemäss Anspruch 1, dadurch gekennzeichnet, dass eine der Chinacridonverbindungen ein unsubstituiertes Chinacridon ist.

4. Verfahren gemäss Anspruch 3, dadurch gekennzeichnet, dass man zwei Chinacridonverbindungen ausgewählt aus der Gruppe bestehend aus unsubstituiertem Chinacridon und durch den gleichen Substituent F, Cl, Br, $CH_3$ oder $OCH_3$ 4,11-disubstituierten Chinacridonen verwendet.

5. Verfahren gemäss Anspruch 3, dadurch gekennzeichnet, dass man zwei Chinacridonverbindungen ausgewählt aus der Gruppe bestehend aus unsubstituiertem Chinacridon und durch den gleichen Substituent F, Cl, Br, $CH_3$ oder $OCH_3$ 2,9-disubstituierten Chinacridonen verwendet.

6. Verfahren gemäss Anspruch 3, dadurch gekennzeichnet, dass man zwei Chinacridonverbindungen ausgewählt aus der Gruppe bestehend aus unsubstituiertem Isochinacridon und symmetrisch durch F, Cl, Br, $CH_3$ oder $OCH_3$ disubstituierten Isochinacridonen verwendet.

7. Verfahren gemäss Anspruch 3, dadurch gekennzeichnet, dass das Verhältnis von unsubstituiertem Chinacridon zu disubstituiertem Chinacridon 95:5 bis 5:95 beträgt.

8. Verfahren gemäss Anspruch 1, dadurch gekennzeichnet, dass der Alkohol Methanol, Ethanol, Butanol, Pentanol oder Ethylenglykol ist.

9. Verfahren gemäss Anspruch 1, dadurch gekennzeichnet, dass der Alkohol Methanol ist.

10. Verfahren gemäss Anspruch 1, dadurch gekennzeichnet, dass die Base Natrium- oder Kaliumhydroxid ist.

11. Verfahren gemäss Anspruch 1, dadurch gekennzeichnet, dass die genannten subpigmentären Chinacridonverbindungen durch Vorvermahlen oder durch Vorbehandlung mit Säure erhalten werden.

12. Verfahren gemäss Anspruch 1, dadurch gekennzeichnet, dass die Mahlung in Gegenwart eines wachstumshemmenden Mittels durchgeführt wird.

13. Verfahren gemäss Anspruch 12, dadurch gekennzeichnet, dass 2-Phthalimidomethylchinacridon als wachstumshemmendes Mittel eingesetzt wird. 14. Verfahren gemäss Anspruch 1, dadurch gekennzeichnet, dass die Partikelgrösse der erhaltenen festen Lösung 0,01 bis 0,8 µ beträgt.